# EUROPEAN PATENT APPLICATION

(11) **EP 1 813 296 A1**
(43) Date of publication of application: **01.08.2007**
(21) Application number: 05719313.8
(22) Date of filing: 18.02.2005
(51) Int. Cl.: A61M 1/14

(54) **AUTOMATIC REINFUSING SYSTEM**

(30) Priority: 19.11.2004 JP 2004335373
(71) Applicant: JMS Co., Hiroshima-shi, Hiroshima 730-0812 (JP)
(72) Inventor: YAMANAKA, Kunihiko c/o JMS CO., 12-17,, 7300812 (JP); MASAOKA, Katsunori c/o JMS CO., 12-17,, 7300812 (JP)
(74) Representative: Hermann, Gerhard
(86) International application number: PCT/JP2005/002644
(87) International publication number: WO 2006/054367

(57) **Abstract**

Provided is an automatic reinfusing system which involves no contamination or medical errors or any other operational problems and which allows easy and simple operation, without imposing any burden on the medical worker. The automatic reinfusing system includes a hemodialysis circuit, **characterized in that** a solution replenishing line has a replenishing solution supply source and an opening and closing means for opening and closing the solution replenishing line, a dialysate supply line and a dialysate discharge line for perfusing to the dialyzer, with solution feeding means being respectively provided for the dialysate supply line and the dialysate discharge line, at least one filtering and reverse- filtering third solution feeding means capable of normal and reverse rotation and allowing flow rate control and serving to perform filtering and reverse filtering on one of the dialysate supply line and the dialysate discharge line in parallel with the solution feeding means, and a water-slowing and solution-discharging means, with the blood feeding means and the filtering and reverse-filtering third solution feeding means being caused to communicate with each other by an interlocking control means for controlling, in an interlocked manner, the blood feeding means, the solution feeding means, the opening and closing means for opening and closing the solution replenishing line, and the opening and closing means between the artery connection end the solution replenishing line branching part.

## Description

### Technical Field

The present invention relates to a hemodialyzer whereby a series of operations including hemodialysis and corresponding blood collection (reinfusion) which were used to be performed manually are automated to a maximal extent for labor savings. In particular, the present invention relates to an automatic reinfusing system.

### Background Art

A hemodialyzer is medical equipment for purifying the blood of renal failure patients and drug intoxication patients. The mechanism for hemodialysis treatment generally is composed of three components: a hemodialyzer, a blood line through which blood circulates, and a dialysate supply system. While maintaining an extracorporeal circulation by means of a blood line which is directly connected to the inside of the blood vessel with two needles (one paracentesis needle in the case of "single-needle" type), the blood is fed into the compartment inside a hollow fiber of the hemodialyzer, which is connected to a certain midpoint in the blood line.

On the other hand, an electrolyte solution called dialysate is flown into the compartment outside the hollow fiber of the hemodialyzer in the opposite direction to the blood flow. Both compartments of the hemodialyzer are separated by a separation membrane called hemodialysis membrane, and while the blood and dialysate flow in opposite directions, material transfer by diffusion occurs depending on the density gradient across the separation membrane, and thus removal of uremic toxin and intoxicating substances as well as replenishing of substances in shortage occur. Generally, the above-mentioned hemodialyzing apparatus is composed of equipment for controlling the maintenance of extracorporeal circulation, stable supply of dialysate, removal of excess water from blood, and the like.

In conventional hemodialysis monitoring apparatuses, the solution replenishing operation during dialysis, the hemodialysis (blood circulation), and the reinfusing operation after the completion of the dialysis are conducted as separate steps; in particular, the reinfusing operation must be performed carefully in the correct order so that no air may not get into the body of the patient, from whose blood vessel the needle has been removed. That is, conventionally, a so-called "air reinfusion" is executed, in which physical saline is poured into the circuit when starting reinfusion and in which after replacement with physical saline has been effected halfway through, reinfusion is conducted while introducing air into the circuit. Further, there is also the risk of air being allowed to be mixed in due to separation of air staying at a stepped portion and a chamber portion of the circuit. Further, when removing the needle on the artery side and pouring physical saline from the artery side circuit end, it may occur that the needle removal is erroneously performed on the vein side first.
As a result, a mental burden is imposed on the medical worker, and the reinfusing operation takes a considerable amount of time.

To reliably perform dialysis and reinfusing operation, Patent Document 1 discloses a method in which main piping connecting a blood outlet and a blood inlet is provided with a replenishing solution supply means connected thereto through the intermediation of a branch pipe, in which there are provided a first clamp for opening and closing the portion of the main piping between the branching point of the branch pipe and the blood outlet, a second clamp for opening and closing the portion of the main piping provided between a second chamber and the blood inlet, and a third clamp provided on the branch pipe and adapted to open and close the branch pipe, and in which a bubble detector is provided in the vicinity of each of the blood outlet and the blood inlet to perform hemodialysis and reinfusion, thereby achieving an improvement in terms of the efficiency of the reinfusing operation.
Patent Document 1: JP 06-261938 A

### Disclosure of the Invention

### Problems to be solved by the Invention

As described above, when performing reinfusing operation after the completion of hemodialysis, there is the risk of bacterial contamination, an operational error in terms of connection/operational-order, etc., and there is a demand for an improvement in this regard. On the other hand, in the conventional method as described above, only physical saline (the liquid medication in the replenishing solution bottle) is considered as the liquid to be poured into the blood circuit, so the pressure in the blood circuit during the reinfusion to the artery side undergoes great fluctuations, so it is impossible to detect any clogging generated in the circuit.
It is accordingly an object of the present invention to provide an automatic reinfusing system which involves no contamination or medical errors or any other operational problems and which allows easy and simple operation, without imposing any burden on the medical worker.

### Means for solving the Problems

In the following description, the components are indicated by the same reference symbols (numerals) as those used in the accompanying drawings; however, this should not be construed restrictively.
According to the present invention, the above-mentioned object is achieved by an automatic reinfusing system including: an artery side (blood) circuit 1 on the upstream side of a blood dialyzer 2; a vein side (blood) circuit 10 on the downstream side thereof; a solution replenishing line 5 branching off from the artery side circuit 1 and serving to perform solution replenishment on the circuit; and a means 18 for opening and closing the solution replenishing line, characterized in that:
the artery side circuit 1 has an artery side connection end A connected with the artery of the patient through a needling means (not shown), an opening/closing means 19 between the artery side connection end A and a solution replenishing line branching part 5a, a dialyzer connection end A' for connection with the blood dialyzer 2, and an artery chamber 4, a blood pump 3, the solution replenishing line branching part 5a, a bubble detecting means 6, and the opening/closing means 19 that are provided between the ends A and A';
the vein side circuit 10 has a dialyzer connection end B' for connection with the blood dialyzer 2, a vein side connection end B, a vein chamber 11 provided between the ends B and B', a bubble detecting means 12, and an opening/closing means 13;
the solution replenishing line 5 has a replenishing solution supply source 7, a means 18 for opening and closing the solution replenishing line, and a pressure sensor 23 as needed; and
there are provided a dialysate supply line 20 and a dialysate discharge line 21 for perfusing to the dialyzer 2, with solution feeding means P2 and P3 being respectively provided for the dialysate supply line 20 and the dialysate discharge line 21, there being provided at least one filtering/reverse-filtering third solution feeding means P1 capable of normal and reverse rotation and allowing flow rate control and serving to perform filtering and reverse filtering on one of the dialysate supply line 20 and the dialysate discharge line 21 in parallel with the solution feeding means, and a water-slowing/solution-discharging means P4, with the blood pump 3 and the filtering/reverse-filtering third solution feeding means P1 being connected to each other by an interlocking control means 16 for controlling, in an interlocked manner, the opening/closing means 13 of the vein side circuit, the replenishing solution opening/closing means 18, and the opening/closing means 19 of the artery circuit 1.

Further, in an automatic reinfusing system according to the present invention, interlock-control can be effected by the interlocking control means according to each step and situation such that the respective flow rates (flow velocities) of the blood pump 3 and the filtering/reverse-filtering third solution feeding means P1 are substantially equalized, or increased, or decreased.
Further, the vein chamber 11 is provided with an overflow line 14 for causing the liquid in the chamber to overflow, and an opening/closing means 15 is provided in the overflow line.

In the solution replenishing line 5, a pressure measuring means P can be provided in a communication circuit between the solution replenishing line branching part 5a and the position of the blood pump 3 or the position of the opening/closing means 18 between the artery side connection end A and the solution replenishing line branching part 5a.
Further, in an automatic reinfusing system according to the present invention, based on the inner pressure of the blood circuit obtained by the pressure measuring means P, it is possible to interlock-control the blood pump 3, the solution feeding means P2 and P3, the filtering/reverse-filtering third solution feeding means P1, the opening/closing means 18 for opening and closing the solution replenishing line, and the opening/closing means between the artery connection end the solution replenishing line branching part.

In the present invention, the upstream side and the downstream side (of the circuit) are based on the flow passage direction (from the artery side to the vein side) when hemodialysis is performed normally, with the blood circuit communicating with the artery/vein of the patient.

In the automatic reinfusing system of the present invention, constructed as described above, when performing hemodialysis , it is possible to perform rapid solution replenishment during hemodialysis by driving the opening/closing means 18 of the solution replenishing line 5 and the opening/closing means 19 of the artery circuit 1 and driving the blood pump 3, and it is possible to automatically effect transition from the hemodialysis step to the reinfusing step by interlock-controlling (adjusting) the blood pump 3, the opening/closing means 18 of the solution replenishing line 5, and the opening/closing means 19 of the artery circuit.
When the dialysis is completed and the reinfusing operation is started, the opening/closing means (19) of the artery circuit is closed in interlock with the opening of the opening/closing means 18 of the solution replenishing line, thus starting the reinfusing operation.
According to the construction of the present invention, when effecting transition from dialysis to reinfusion step, there is no need to perform the operation of removing the needle from the body of the patient and attaching/detaching the circuit in the transition from hemodialysis to the reinfusing operation, so it is possible to prevent errors in terms of needle removal order and attachment/detachment position. That is, in the conventional reinfusing operation, reinfusion is effected by supplying blood to the vein side only, whereas, in the present invention, reinfusion is effected in the directions of both the artery and the vein, so there is no need to effect needle removal until the reinfusion step has been completed.
Further, the bubble detecting means 6 and 12 are attached to both the artery side circuit 1 and the vein side circuit 10, whereby it is possible to prevent bubbles from flowing into the blood vessels of the patient.

Further, by controlling, in an interlocked manner, the respective flow rates at the blood pump 3, the opening/closing means 18 of the solution replenishing line 5, and the filtering/reverse-filtering third solution feeding means P1, and the opening/closing means 12 of the vein side circuit 10, it is possible to control the flowing directions and flow rates of the blood and the replenishing solution in the circuit. As a result, the transition from the hemodialysis step to the reinf using operation can be effected easily, quickly, and reliably.

### Effects of the Invention

In the automatic reinfusing system of the present invention, the solution replenishing operation during hemodialysis and the reinfusing operation after the completion of dialysis can be conducted safely without involving any contamination or operational errors; further, the operation which has required manual labor can be automated, thereby achieving an improvement in operational efficiency and labor saving. Further, due to the automation and simplification of the operation, there is no need for the medical worker to be particularly experienced or skilled as in the prior art.

### Brief Description of the Drawing

Fig. 1 is a schematic view of an example of the automatic reinfusing system of the present invention, illustrating it in the hemodialysis step.
Fig. 2 is a schematic view illustrating how solution replenishment is effected during hemodilysis in the system of Fig. 1.
Fig. 3 is a schematic view of an example of the automatic reinfusing system of the present invention, illustrating an automatic reinfusing step according to an embodiment.
Fig. 4 is a schematic view of an example of the automatic reinfusing system of the present invention, illustrating an automatic reinfusing step according to another embodiment.
Fig. 5 is a schematic view of an example of the automatic reinfusing system of the present invention, illustrating how reinfusion is effected simultaneously on an artery side circuit and a vein side circuit.

### Description of the Reference Symbols

An explanation of the mark is as follows:
1. artery side (blood) circuit, 2. blood dialyzer, 3. blood pump, 4. artery chamber, 5. solution replenishing line, 5A. solution replenishing line branching part, 6. bubble sensor, 7. replenishing solution container, 8. replenishing solution pump, 9. pressure sensor, 10. vein side circuit, 11. vein chamber, 12. bubble sensor, 13. clamp, 14. overflow line, 15. clamp, 16. control device, 17. communication passage, 18. solution replenishing line clamp, 19. opening/closing means, 20. dialysate supply line, 21. dialysate discharge line, 22. dialysate filtering/reverse-filtering line, 23. pressure sensor, P1. filtering/reverse-filtering pump, P2. dialysate feeding pump, P3. dialysate discharging pump, P4. water slowing pump, A. artery side connection end, A': dialyzer artery side connection end, B. vein side connection end, B': dialyzer vein side connection end

### Best Mode for carrying out the Invention

Further, in the present invention, it is possible to obtain specific effects by the various embodiments described below. That is, in the above-mentioned automatic reinfusing system, the vein side circuit 10 has an opening/closing means 13 for opening and closing the vein side circuit, and the interlocking control means 16 communicates with the opening/closing means 13, making it also possible to control the opening/closing means 13 in an interlocked manner.
In the automatic reinfusing system of the present invention, the regulation and selection of the flow passages is made more reliable and safer by adjusting the ratio of the flow rates at the opening/closing means 18 of a solution replenishing line 5 and the filtering/reverse-filtering third solution feeding means P1 and the blood feeding flow rate of a blood pump 3 or the solution feeding directions.

Further, in the automatic reinfusing system of the present invention, it is possible to effect interlocking control with the interlocking control means 16, according to each step and situation, such that the respective flow rates (flow velocities) at both the blood pump 3 and the filtering/reverse-filtering third solution feeding means P1 are substantially equalized, or increased, or decreased. With the above-mentioned construction, it is possible to freely change the reinfusing direction and order and the reinfusing mode (i.e., simultaneously on the artery and the vein or sequentially one at a time), thereby making it possible to recover the valuable blood without wasting it.

Further, in the automatic reinfusing system of the present invention as described above, the vein chamber 11 is provided with an overflow line 14 for effecting overflow of the liquid in the chamber, with an opening/closing means 15 being attached to the overflow line 14.
With the above-mentioned construction, while pouring in the replenishing solution at the time of priming, a portion of the solution can be caused to flow out of the overflow line 14, thus making it possible to effect efficient cleaning and bubble removal.

Further, in the automatic reinfusing system of the present invention as described above, there is provided, in the solution replenishing line 5, a pressure measuring means 23 in a communication circuit between the solution replenishing line branching part 5a and the position of the blood pump 3 or the position of an opening/closing means 19 between an artery side connection end A and the solution replenishing line branching part 5a. By providing the pressure measuring means 23 at the above-mentioned position in the solution replenishing line 5, it is possible to prevent any abnormal increase or reduction in pressure and to estimate the current solution feeding state, which proves effective.

### Embodiment 1

In the following, an embodiment of the present invention will be described in more detail.

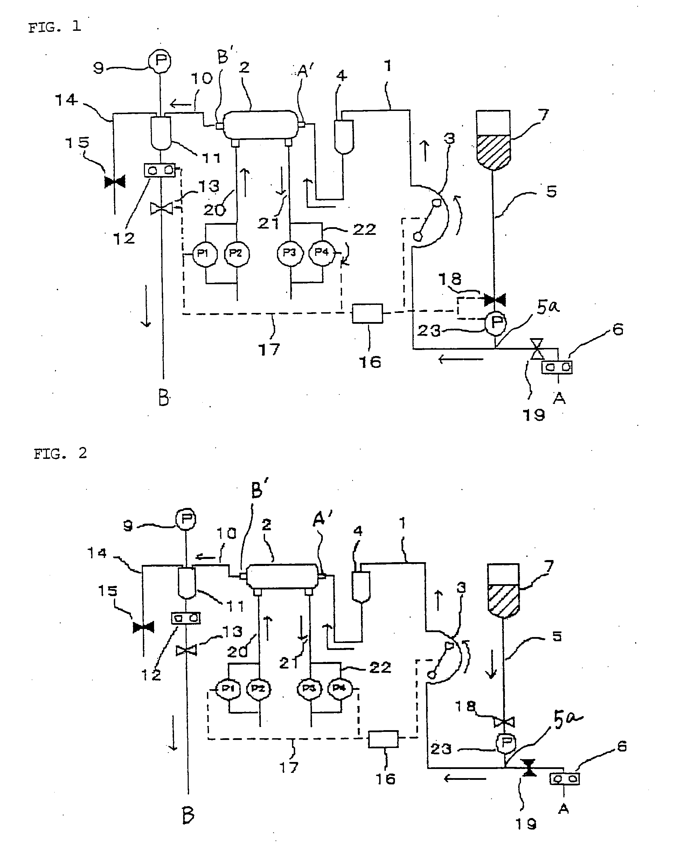

Figs. 1 through 5 schematically show the construction and arrangement of the automatic reinfusing system and the flow passages and flowing directions of the blood and the replenishing solution in each step and in each state. Fig. 1 is a schematic view showing how hemodialysis is conducted without performing solution replenishment. In Fig. 1, the artery side (blood) circuit 1 starts at the artery side connection end A, and terminates at the dialyzer side connection end A' connected to a blood dialyzer 2. In the artery side circuit 1, there are provided the blood pump 3 and an artery chamber 4 in that order from the upstream side; between the blood pump 3 and the artery side connection end A, there exists the solution replenishing line branching part 5a connected to the solution replenishing line 5. A bubble sensor 6 is provided between the branching part 5a and the artery side connection end A. The opening/closing means 19 is provided between the solution replenishing line branching part 5a and the artery connection end A.

The solution replenishing line 5 starts at a replenishing solution container 7 serving as a supply source of the replenishing solution and terminates at the solution replenishing line branching part 5a, with the opening/closing means 18 being provided in the solution replenishing line 5. There is provided the pressure sensor 23 for measuring the pressure between the opening/closing means 18 in the solution replenishing line and the branching part 5a.

A vein side (blood) circuit 10 starts at a dialyzer 2 connection end B' and terminates at a vein side connection end B. In the vein side circuit 10, there are provided the vein chamber 11, a bubble sensor 12, and a clamp 13 constituting the opening/closing means, in that order from the upstream side.
Connected to the upper portion of the vein chamber 11 is the overflow line 14, making it possible to effect overflow and removal of cleaning liquid and bubbles at the time of priming of the blood circuit. Attached to the overflow line 14 is a clamp 15 capable of opening and closing the line 14.

The blood pump 3, the opening/closing means 18 of the solution replenishing line 5, the opening/closing means 19 of the artery circuit 1, and the filtering/reverse-filtering third solution feeding means P1 provided in parallel with the dialysate perfusing line are allowed to communicate with the control device 16 through a communication passage 17, and the blood pump 3 and the filtering/reverse-filtering third solution feeding means P1 are not only driven and stopped by the control device 16 but also controlled in an interlocked manner such that predetermined values are attained in their respective solution feeding directions and solution feeding amounts (flow velocities) or that the ratio of the respective solution feeding amounts of the blood pump 3 and the filtering/reverse-filtering third solution feeding means P1 attains a predetermined value. Further, the control device 16 communicates through the communication passage 17 so as to be capable of an interlocking control of the opening and closing of the clamp 13 attached to the vein side circuit 10, the driving/stopping and the flow rate adjustment of the blood pump 3 and the filtering/reverse-filtering third solution feeding means P1.

In the following, a brief description will be given of each step and how the blood feeding and solution feeding for the blood circuit are performed in that step.
Fig. 1 shows how hemodialysis is effected without any solution replenishing operation. The opening/closing means 18 of the solution replenishing line 5 is closed, and the opening/closing means 19 of the artery circuit 1 is open, with solely the blood pump 3 being run. The clamp 13 is open, and the clamp 15 is closed. The opening/closing means 18 of the solution replenishing line 5 is closed, and no blood flows through the solution replenishing line 5; as indicated by arrows of Fig. 1, the blood flowing into the artery side circuit 1 from the artery connection end A passes through the blood dialyzer 2 and the vein side circuit 10 before being returned to the patient from the vein side connection end B.

Here, the clamp 15 is closed, and no liquid flow occurs in the overflow line 14. Further, in all of the hemodialysis step, the solution replenishing step, and the reinfusing step described in connection with this embodiment, the overflow line 14 remains closed 14. The same construction is adopted for each step.

Next, the solution replenishing step will be described. When, during hemodialysis, it is necessary to perform rapid solution replenishment because of a reduction in the blood pressure, etc. of the patient, the opening/closing means 18 of the solution replenishing line 5 is opened, and the opening/closing means 19 of the artery circuit 1 is closed, as shown in Fig. 2. The solution replenishment is controlled based on the flow velocity at the blood pump 3. For example, when the flow velocity at the blood pump 3 is 100 ml/min, the flow velocity of the replenishing solution is 100 ml/min. When the flow velocity at the blood pump 3 is 100 ml/min and the opening/closing means 19 of the artery circuit 1 is closed, the flow velocity of the blood flowing in at the artery side connection end A is 0 ml/min, and the replenishing solution flowing out of the solution replenishing line 5 flows through the blood circuit. Further, by setting the solution replenishing time, it is possible to determine the solution replenishing amount.

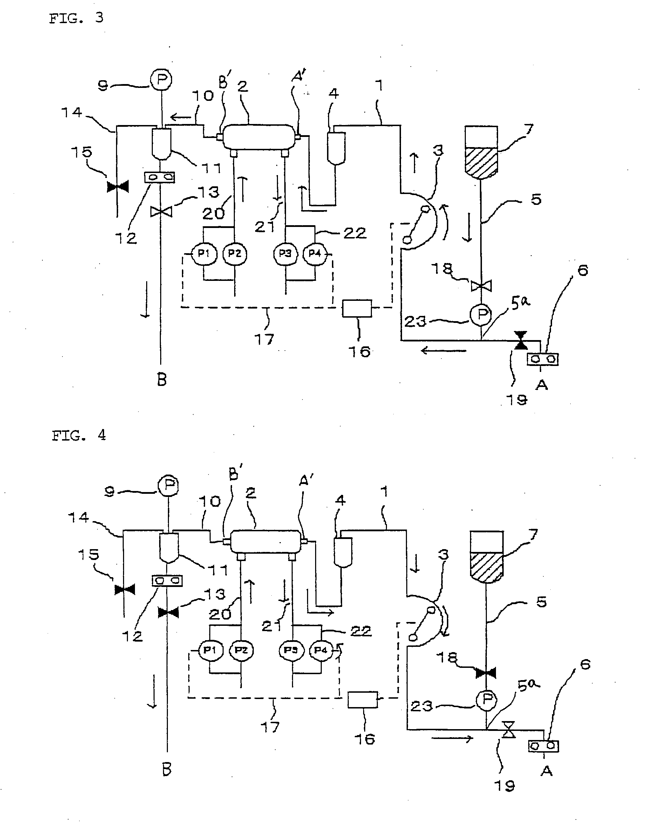

Next, the reinfusing step will be described. The control in the reinfusing step varies depending on the method; here, only a typical control procedure will be described. In an example of the control mode, the blood filling the portion of the blood circuit 1 on the downstream side of the solution replenishing line branching part 5a is first reinfused (Fig. 3), and then the blood filling the portion of the blood circuit on the upstream side of the solution replenishing line branching part 5a is returned (Fig. 4).

In Fig. 3, control is effected such that the opening/closing means 18 of the solution replenishing line is open and that the opening/closing means 19 of the artery circuit 1 is closed, and the blood filling the portion of the blood circuit on the downstream side of the solution replenishing line branching part 5a is returned at the flow velocity at the blood pump 3 to the patient as indicated by arrows of Fig. 3. At the point in time when the blood completely flows out of the portion of the blood circuit 1 on the downstream side of the solution replenishing line branching part 5a, control is effected such that the opening/closing means 18 of the solution replenishing line 5 is closed, and that the opening/closing means 19 of the artery circuit is opened; in interlock with the switching thereof (i.e., in interlock with the switching of the opening/closing means 18 to the closed state and the switching of the opening/closing means 19 to the open state: If this switching is not completed (i.e., if the switching is not normally effected), the reverse rotation of the blood pump 3 and the operation in the reverse filtering direction of the filtering/reverse-filtering third solution feeding means P1 are not conducted), the blood pump 3 is caused to make reverse rotation, and at the same time, the filtering/reverse-filtering third solution feedingmeans (filtering/reverse-filtering pump) P1 of the dialysate line is operated in the reverse filtering direction. Then, as shown in Fig. 4, the blood remaining on the upstream side of the branching line 5 is returned to the patient from the artery side connection end A by the reverse-filtered liquid from the dialyzer 2.

At this time, in Fig. 4, the clamp 13 may be interlock-controlled by the control device 16 to be closed.
Further, in this reinfusing step, the blood pump 3 and the filtering/reverse-filtering third solution feeding means P1 are controlled by the pressure as measured by the pressure measuring means (pressure sensor 9 or pressure sensor 23) to prevent an abnormal increase in the pressure inside the blood circuit, or to issue a warning upon an abnormal increase in pressure, and at the same time, effect closing by the opening/closing means, etc, thus making it possible to secure safety.

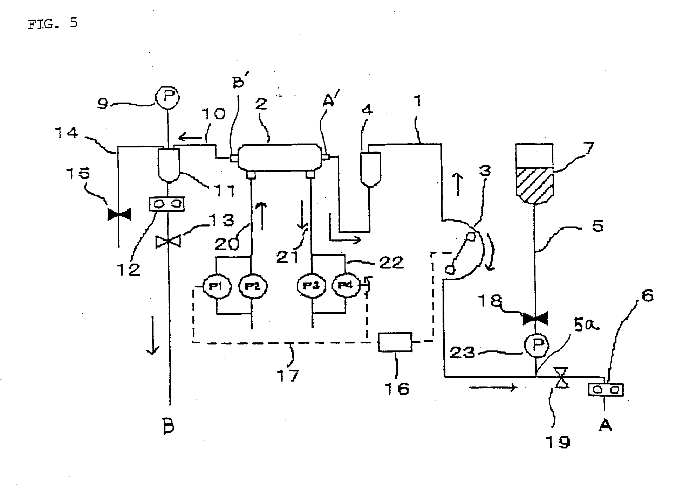

Fig. 5 shows another example of the control mode. That is, the flow velocity at the filtering/reverse-filtering third solution feeding means P1 of the dialysate perfusing line is made higher than the flow velocity at the blood pump 3 making reverse rotation, whereby it is also possible to simultaneously return to the patient the blood remaining on the upstream side and the downstream side of the solution replenishing line branching part 5a. That is, due to the filtering/reverse-filtering third solution feeding means P1, of the reverse-filtered liquid from the dialyzer 2, an amount of liquid equal to that of the blood pump 3 is caused to flow through the artery side blood circuit 1, and the remaining (surplus) liquid flows through the blood circuit 10 to be returned to the patient. In this method, the reinfusion can be effected simultaneously through the artery side connection end A and the vein side connection end B, so the operation can be completed in a shorter period of time.

### Industrial Applicability

The automatic reinfusing system of the present invention is suitable for an automatic reinfusing system which involves no contamination or medical errors in the solution replenishing operation during hemodialysis and in the reinfusing operation after the completion of the dialysis, and which allows a highly reliable reinfusing operation imposing no burden on the medical worker.

## Claims

1. An automatic reinfusing system, comprising:
an artery side (blood) circuit on an upstream side of a blood dialyzer;
a vein side (blood) circuit on a downstream side thereof; and
a solution replenishing line branching off from the artery side circuit and serving to perform solution replenishment on the circuit, **characterized in that**:
the artery side circuit has an artery side connection end connected with an artery of a patient through a needling means, a dialyzer connection end for connection with a blood dialyzer, and an artery chamber, a blood feeding means, a solution replenishing line branching part, and a bubble detecting means that are provided between the two ends, and an opening and closing means provided between the artery side connection end and the solution replenishing line branching part;,
the vein side circuit has the dialyzer connection end for connection with the blood dialyzer, a vein side connection end, a vein chamber, a bubble detecting means, and an opening and closing means that are provided between the two ends;
the solution replenishing line has a replenishing solution supply source and an opening and closing means for opening and closing the solution replenishing line; and
the blood feeding means (blood pump) provided on the artery circuit side of this bloodcirculation systemhas a blood pump allowing flow rate control and capable of normal and reverse rotation, there being further provided a dialysate supply line and a dialysate discharge line for perfusing to the dialyzer, with solution feeding means being respectively provided for the dialysate supply line and the dialysate discharge line, there being provided at least one filtering and reverse-filtering third solution feeding means capable of normal and reverse rotation and allowing flow rate control and serving to perform filtering and reverse filtering on one of the dialysate supply line and the dialysate discharge line in parallel with the solution feeding means, and a water-slowing and solution-discharging means, with the blood feeding means and the filtering and reverse-filtering third solution feeding means being caused to communicate with each other by an interlocking control means for controlling, in an interlocked manner, the blood feeding means, the solution feeding means, the opening and closing means for opening and closing the solution replenishing line, and the opening and closing means between the artery connection end the solution replenishing line branching part.

2. An automatic reinfusing system according to Claim 1, **characterized in that** the vein side circuit has an opening and closing means for opening and closing the vein side circuit, and that the interlocking control means is caused to communicate with the opening and closing means to make it also possible to interlock-control the opening and closing means.

3. An automatic reinfusing system according to Claim 1 or 2, **characterized in that** interlock-control can be effected by the interlocking control means according to each step and situation such that the respective flow rates (flow velocities) of the blood feeding means and the filtering and reverse-filtering third solution feeding means are substantially equalized, or increased, or decreased.

4. An automatic reinfusing system according to one of Claims 1 through 3, **characterized in that**:
the vein chamber is provided with an overflow line for causing the liquid in the chamber to overflow; and
an opening and closing means is provided in the overflow line.

5. An automatic reinfusing system according to one of Claims 1 through 4, **characterized in that**, in the solution replenishing line, a pressure measuring means is provided in a communication circuit between the solution replenishing line branching part and the position of the blood feeding means or the position of the opening and closing means between the artery side connection end and the solution replenishing line branching part.

6. An automatic reinfusing system according to one of Claims 1 through 5, **characterized in that**, based on the inner pressure of the blood circuit obtained by the pressure measuring means, the blood feeding means, the solution feeding means, the filtering and reverse-filtering third solution feeding means, the opening and closing means for opening and closing the solution replenishing line, and the opening and closing means between the artery connection end the solution replenishing line branching part can be interlock-controlled.
